# EUROPEAN PATENT SPECIFICATION

(11) **EP 3 824 796 B1**
(45) Date of publication and mention of the grant of the patent: **15.05.2024**
(21) Application number: 19837783.0
(22) Date of filing: 17.06.2019
(51) Int. Cl.: A61B 1/00, A61B 1/06

(54) **ENDOSCOPE SYSTEM**
ENDOSKOPSYSTEM
SYSTÈME D'ENDOSCOPE

(30) Priority: 20.07.2018 JP 2018136923
(43) Date of publication of application: 26.05.2021
(73) Proprietor: FUJIFILM Corporation, Tokyo 106-8620 (JP)
(72) Inventor: USUDA, Toshihiro, Ashigarakami-gun, Kanagawa 258-8538 (JP)
(74) Representative: Kudlek, Franz Thomas
(86) International application number: PCT/JP2019/023883
(87) International publication number: WO 2020/017212

(56) References cited:
- EP-A1- 3 216 381
- EP-A1- 3 795 062
- WO-A1-2018/105063
- JP-A- 2008 301 968
- JP-A- 2010 172 673
- JP-A- 2011 200 283
- JP-A- 2012 245 161
- US-A1- 2011 275 889
- US-A1- 2018 114 319

## Description

### BACKGROUND OF THE INVENTION

### 1. Field of the Invention

The present invention relates to an endoscope system and specifically relates to a technique for detecting a region of interest from an endoscopic image and giving a notification.

### 2. Description of the Related Art

During an endoscopic examination, basically, a doctor inserts a scope into the interior of an organ while washing off dirt adhered to the organ, and thereafter, withdraws the scope while observing the inside of the organ. The operations by a doctor during insertion and those during withdrawal are different. Accordingly, an endoscope apparatus that operates differently during insertion and during withdrawal is available.

For example, WO2017/006404A discloses a technique for making the frame rate of an image capturing unit in a case where the movement direction of an insertion part is an insertion direction higher than in a case where the movement direction is a withdrawal direction. With this technique, even when an image changes frequently due to shaking or moving of the distal end of the insertion part during insertion, the image can be displayed on an image display unit as a smooth moving image, and the insertion operation can be smoothly performed. US 2018/0114319 A1 relates to an endoscope system including an endoscope and an image processing device attached to one another, wherein the image processing device includes at least one processor configured to perform operations of determining an operator's action based on an action signal from an endoscope inserted into a subject body, deciding whether an image is set as a detection target image based on the operator's action and detecting a specific region from the detection target image.

### SUMMARY OF THE INVENTION

As a technique for assisting a doctor who is performing an endoscopic examination, a technique for detecting a region of interest, such as a lesion, from an endoscopic image and giving a notification is available. However, depending on the timing in the endoscopic examination, the notification of the result of detection of the region of interest can hinder the doctor's operation.

For example, during withdrawal of the insertion part, a lesion needs to be detected as a matter of course, and therefore, a notification of the result of detection that is given to provide assistance is useful. On the other hand, insertion is technically difficult and requires concentration, and therefore, a notification of the result of detection can hinder the doctor's operation during insertion.

According to the invention, provided is an endoscope system for performing an examination of a lumen of a patient according to the independent claim; dependent claims relate to preferred embodiments. The present invention has been made in view of the above-described circumstances, and an object thereof is to provide an endoscope system that appropriately gives a notification of the result of detection of a region of interest.

To achieve the above-described object, the endoscope system according to one aspect is an endoscope system for performing an examination of a lumen of a patient, the endoscope system including: an insertion part that is inserted into the lumen; a camera that performs image capturing of the lumen to obtain an endoscopic image; a region-of-interest detection unit that detects a region of interest from the endoscopic image; a detection result notification unit that gives a notification of a result of detection of the region of interest; an insertion-withdrawal determination unit that determines whether a step in the examination is an insertion step in which the insertion part is inserted up to a return point in the lumen or a withdrawal step in which the insertion part is withdrawn from the return point; and a notification control unit that causes the detection result notification unit to give the notification in accordance with the step determined by the insertion-withdrawal determination unit.

According to this aspect, it is determined whether the step in the examination is the insertion step or the withdrawal step, and a notification of the result of detection of the region of interest is given in accordance with the determined step. Therefore, a notification of the result of detection of the region of interest can be appropriately given.

Preferably, the detection result notification unit includes a display notification unit that gives a notification of detection of the region of interest by display on a display, and the notification control unit causes the display notification unit to display text having a first size in the insertion step, and causes the display notification unit to display text having a second size larger than the first size in the withdrawal step. Accordingly, in the withdrawal step, a notification that is more noticeable than in the insertion step can be given.

Preferably, the detection result notification unit includes a display notification unit that gives a notification of detection of the region of interest by display on a display, and the notification control unit causes the display notification unit to display an icon having a first size in the insertion step, and causes the display notification unit to display an icon having a second size larger than the first size in the withdrawal step. Accordingly, in the withdrawal step, a notification that is more noticeable than in the insertion step can be given.

Preferably, the detection result notification unit includes a display notification unit that gives a notification of detection of the region of interest by display on a display, and the notification control unit causes the display notification unit to display a background in a first background color in the insertion step, and causes the display notification unit to display a background in a second background color higher in brightness than the first background color in the withdrawal step. Accordingly, in the withdrawal step, a notification that is more noticeable than in the insertion step can be given.

Preferably, the detection result notification unit includes a display notification unit that gives a notification of detection of the region of interest by display on a display, and the notification control unit causes the display notification unit to display a frame having a first size and including the region of interest together with the endoscopic image in the insertion step, and causes the display notification unit to display a frame having a second size larger than the first size and including the region of interest together with the endoscopic image in the withdrawal step. Accordingly, in the withdrawal step, a notification that is more noticeable than in the insertion step can be given.

Preferably, the detection result notification unit includes a display notification unit that gives a notification of detection of the region of interest by display on a display, and the notification control unit causes the display notification unit to hide or display an icon in the insertion step, and causes the display notification unit to superimpose on the endoscopic image and display a geometric shape that indicates an area of the region of interest at a position of the region of interest in the withdrawal step. Accordingly, in the withdrawal step, a notification that is more noticeable than in the insertion step can be given.

The detection result notification unit includes a display notification unit that gives a notification of detection of the region of interest by display on a display, and the notification control unit causes the display notification unit to superimpose on the endoscopic image and display a geometric shape that indicates an area of the region of interest at a position of the region of interest only at a time of detection of the region of interest in the insertion step, and causes the display notification unit to superimpose on the endoscopic image and display the geometric shape that indicates the area of the region of interest at the position of the region of interest at the time of detection of the region of interest and keep displaying the geometric shape for a certain period after the detection in the withdrawal step. Accordingly, in the withdrawal step, a notification that is more noticeable than in the insertion step can be given.

Preferably, the detection result notification unit includes a sound notification unit that gives a notification of detection of the region of interest by outputting a sound, and the notification control unit causes the sound notification unit to output the sound at a first sound volume in the insertion step, and causes the sound notification unit to output the sound at a second sound volume higher than the first sound volume in the withdrawal step. Accordingly, in the withdrawal step, a notification that is more noticeable than in the insertion step can be given.

Preferably, the detection result notification unit includes a lighting notification unit that gives a notification of detection of the region of interest by lighting a lamp, and the notification control unit causes the lighting notification unit to light the lamp with a first amount of light in the insertion step, and causes the lighting notification unit to light the lamp with a second amount of light larger than the first amount of light in the withdrawal step. Accordingly, in the withdrawal step, a notification that is more noticeable than in the insertion step can be given.

Preferably, the detection result notification unit includes a display notification unit that gives a notification by display on a display, and a sound notification unit that gives a notification by outputting a sound, and the notification control unit causes the display notification unit to superimpose on the endoscopic image and display a geometric shape that indicates an area of the region of interest at a position of the region of interest in the insertion step, and causes the display notification unit to superimpose on the endoscopic image and display the geometric shape that indicates the area of the region of interest at the position of the region of interest and causes the sound notification unit to output the sound in the withdrawal step. Accordingly, in the withdrawal step, a notification that is more noticeable than in the insertion step can be given.

Preferably, the detection result notification unit includes a display notification unit that gives a notification by display on a display, and a sound notification unit that gives a notification by outputting a sound, and the notification control unit causes the sound notification unit to output the sound in the insertion step, and causes the sound notification unit to output the sound and causes the display notification unit to display an icon in the withdrawal step. Accordingly, in the withdrawal step, a notification that is more noticeable than in the insertion step can be given.

Preferably, the detection result notification unit includes a display notification unit that gives a notification by display on a display, a sound notification unit that gives a notification by outputting a sound, and a lighting notification unit that gives a notification by lighting a lamp, and the notification control unit causes the lighting notification unit to light the lamp in the insertion step, and causes the lighting notification unit to light the lamp, causes the display notification unit to superimpose on the endoscopic image and display a geometric shape that indicates an area of the region of interest at a position of the region of interest, and causes the sound notification unit to output the sound in the withdrawal step. Accordingly, in the withdrawal step, a notification that is more noticeable than in the insertion step can be given.

Preferably, the detection result notification unit includes a display notification unit that gives a notification by display on a display, a sound notification unit that gives a notification by outputting a sound, and a lighting notification unit that gives a notification by lighting a lamp, and when N is an integer from 0 to 2, the notification control unit causes N units among the display notification unit, the sound notification unit, and the lighting notification unit to give a notification in the insertion step, and causes at least (N+1) units among the display notification unit, the sound notification unit, and the lighting notification unit to give a notification in the withdrawal step. Accordingly, in the withdrawal step, a notification that is more noticeable than in the insertion step can be given.

According to the present invention, a notification of the result of detection of a region of interest can be appropriately given.

### BRIEF DESCRIPTION OF THE DRAWINGS

Fig. 1 is an external view of an endoscope system;
Fig. 2 is a block diagram illustrating the internal configuration of the endoscope system;
Fig. 3 is a graph illustrating the intensity distributions of light;
Fig. 4 is a block diagram illustrating the configuration of an image recognition unit;
Fig. 5 is a block diagram illustrating another form of the configuration of the image recognition unit;
Fig. 6 is a flowchart illustrating processes of a method for notification of recognition results by the endoscope system;
Fig. 7 is a block diagram illustrating the configuration of a recognition result notification unit according to a first embodiment;
Fig. 8 includes diagrams illustrating examples where a notification of detection of a lesion from an image is given by displaying text;
Fig. 9 includes diagrams illustrating examples where a notification of detection of a lesion from an image is given by displaying an icon;
Fig. 10 includes diagrams illustrating examples where a notification of detection of a lesion from an image is given by displaying a background;
Fig. 11 includes diagrams illustrating examples where a notification of detection of a lesion from an image is given by displaying a frame;
Fig. 12 includes diagrams illustrating examples where a notification of detection of a lesion from an image is given by display that differs depending on the step determined by an insertion-withdrawal determination unit;
Fig. 13 includes diagrams illustrating examples where a notification of detection of a lesion from an image is given by display for different display periods;
Fig. 14 is a block diagram illustrating the configuration of the recognition result notification unit according to a second embodiment;
Fig. 15 is a block diagram illustrating the configuration of the recognition result notification unit according to a third embodiment;
Fig. 16 is a block diagram illustrating the configuration of the recognition result notification unit according to fourth and fifth embodiments; and
Fig. 17 is a block diagram illustrating the configuration of the recognition result notification unit according to a sixth embodiment.

### DESCRIPTION OF THE PREFERRED EMBODIMENTS

Hereinafter, preferred embodiments of the present invention will be described in detail with reference to the attached drawings.

A description is given of a case where the present invention is applied to a lower gastrointestinal endoscope that is inserted through the anus of a subject and used to examine (observe) the lumina of, for example, the rectum and the large intestine. Note that the present invention is applicable to an upper gastrointestinal endoscope that is inserted through the mouse or nose of a patient and used to observe the lumina of, for example, the esophagus and the stomach.

### Configuration of Endoscope System

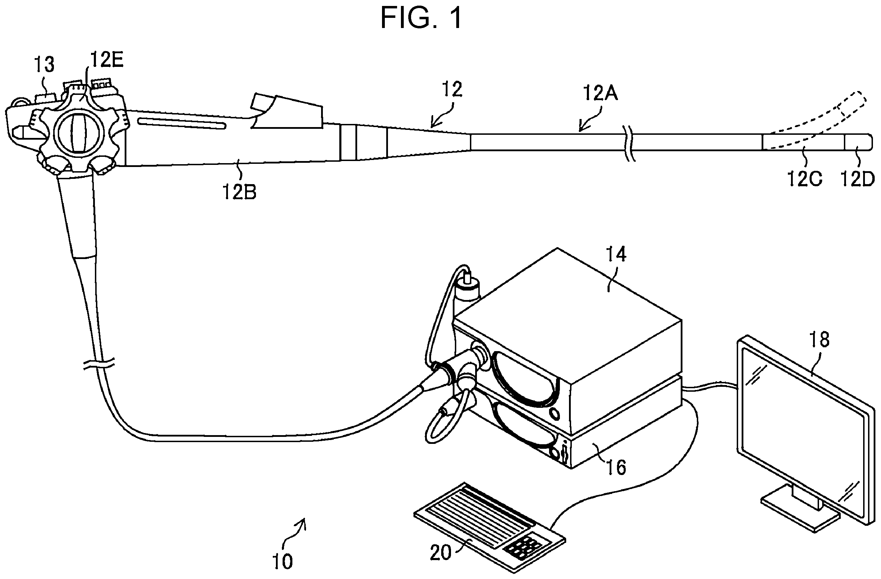

Fig. 1 is an external view of an endoscope system 10. As illustrated in Fig. 1, the endoscope system 10 includes, an endoscope 12, a light source device 14, a processor device 16, a display unit 18, and an input unit 20.

The endoscope 12 is optically connected to the light source device 14. The endoscope 12 is electrically connected to the processor device 16.

The endoscope 12 has an insertion part 12A that is inserted into a lumen of a subject, an operation part 12B that is provided on the proximal end part of the insertion part 12A, and a bending part 12C and a tip part 12D that are provided on the distal end side of the insertion part 12A.

On the operation part 12B, an angle knob 12E and a mode switching switch 13 are provided.

An operation of the angle knob 12E results in a bending operation of the bending part 12C. This bending operation makes the tip part 12D point in a desired direction.

The mode switching switch 13 is used in a switching operation for an observation mode. The endoscope system 10 has a plurality of observation modes in which the wavelength patterns of irradiation light are different. A doctor (operator) can operate the mode switching switch 13 to set a desired observation mode. The endoscope system 10 generates and displays on the display unit 18 an image corresponding to the set observation mode in accordance with the combination of the wavelength pattern and image processing.

On the operation part 12B, an obtaining instruction input unit not illustrated is provided. The obtaining instruction input unit is an interface for a doctor to input an instruction for obtaining a still image. The obtaining instruction input unit accepts an instruction for obtaining a still image. The instruction for obtaining a still image accepted by the obtaining instruction input unit is input to the processor device 16.

The processor device 16 is electrically connected to the display unit 18 and to the input unit 20. The display unit 18 is a display device that outputs and displays, for example, an image of an observation target region and information concerning the image of the observation target region. The input unit 20 functions as a user interface for accepting operations of inputting, for example, functional settings of the endoscope system 10 and various instructions.

The steps in an examination in the endoscope system 10 include an insertion step and a withdrawal step. The insertion step is a step in which the tip part 12D of the insertion part 12A of the endoscope 12 is inserted from an insertion start point up to a return point in a lumen of a patient, and the withdrawal step is a step in which the tip part 12D is withdrawn from the return point up to the insertion start point in the lumen of the patient.

The insertion start point is an end part of the lumen at which insertion of the tip part 12D starts. The insertion start point is, for example, the anus in a case of a lower gastrointestinal endoscope, or the mouse or nose in a case of an upper gastrointestinal endoscope. The return point is the furthest position in the lumen that the tip part 12D reaches. At the return point, a part of the insertion part 12A that is inserted into the lumen becomes largest.

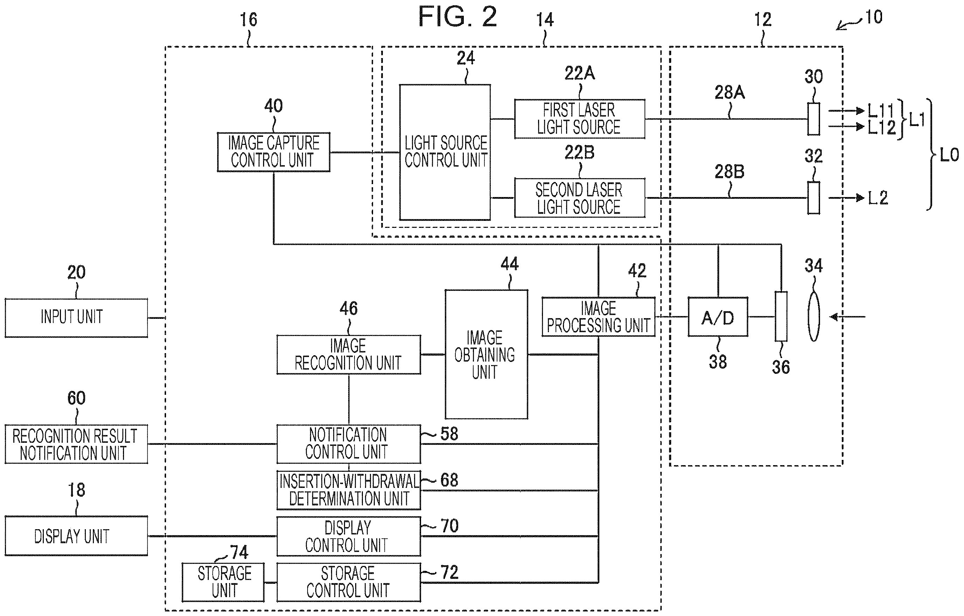

Fig. 2 is a block diagram illustrating the internal configuration of the endoscope system 10. As illustrated in Fig. 2, the light source device 14 includes a first laser light source 22A, a second laser light source 22B, and a light source control unit 24.

The first laser light source 22A is a blue laser light source having a center wavelength of 445 nm. The second laser light source 22B is a violet laser light source having a center wavelength of 405 nm. As the first laser light source 22A and the second laser light source 22B, laser diodes can be used. Light emission of the first laser light source 22A and that of the second laser light source 22B are separately controlled by the light source control unit 24. The ratio between the light emission intensity of the first laser light source 22A and that of the second laser light source 22B is changeable.

As illustrated in Fig. 2, the endoscope 12 includes an optical fiber 28A, an optical fiber 28B, a fluorescent body 30, a diffusion member 32, an image capturing lens 34, an imaging device 36, and an analog-digital conversion unit 38.

The first laser light source 22A, the second laser light source 22B, the optical fiber 28A, the optical fiber 28B, the fluorescent body 30, and the diffusion member 32 constitute an irradiation unit.

Laser light emitted from the first laser light source 22A passes through the optical fiber 28A and irradiates the fluorescent body 30 disposed in the tip part 12D of the endoscope 12. The fluorescent body 30 is formed of a plurality of types of fluorescent bodies that absorb part of blue laser light emitted from the first laser light source 22A, are excited, and emit green to yellow light. Accordingly, light emitted from the fluorescent body 30 is white (pseudo white) light L1 that is a combination of excitation light L11 of green to yellow generated from blue laser light, which is excitation light, emitted from the first laser light source 22A and blue laser light L12 that passes through the fluorescent body 30 without being absorbed.

Note that white light described here is not limited to light that completely includes all wavelength components of visible light. For example, the white light may be light that includes light in specific wavelength ranges of, for example, R (red), G (green), and B (blue). It is assumed that the white light includes, for example, light that includes wavelength components of green to red or light that includes wavelength components of blue to green in a broad sense.

Laser light emitted from the second laser light source 22B passes through the optical fiber 28B and irradiates the diffusion member 32 disposed in the tip part 12D of the endoscope 12. As the diffusion member 32, for example, a translucent resin material can be used. Light emitted from the diffusion member 32 is light L2 having a narrow-band wavelength with which the amount of light is homogeneous within an irradiation region.

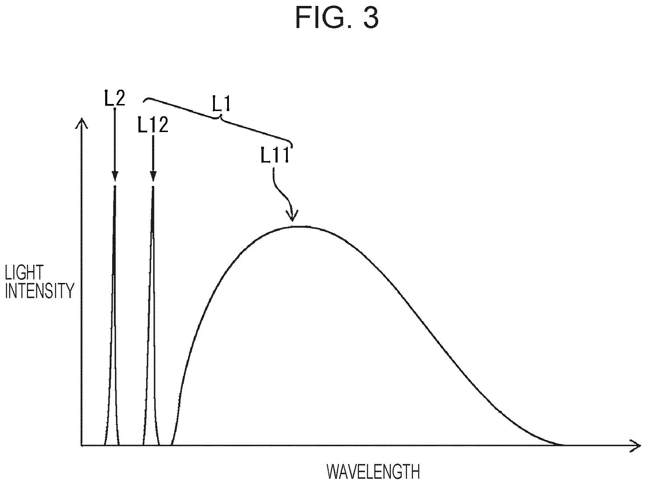

Fig. 3 is a graph illustrating the intensity distributions of the light L1 and the light L2. The light source control unit 24 changes the ratio between the amount of light of the first laser light source 22A and that of the second laser light source 22B. Accordingly, the ratio between the amount of light of the light L1 and that of the light L2 is changed, and the wavelength pattern of irradiation light L0, which is composite light generated from the light L1 and the light L2, is changed. Therefore, the irradiation light L0 having a wavelength pattern that differs depending on the observation mode can be emitted.

Referring back to Fig. 2, the image capturing lens 34, the imaging device 36, and the analog-digital conversion unit 38 constitute an image capturing unit (camera). The image capturing unit is disposed in the tip part 12D of the endoscope 12.

The image capturing lens 34 forms an image of incident light on the imaging device 36. The imaging device 36 generates an analog signal that corresponds to the received light. As the imaging device 36, a CCD (charge-coupled device) image sensor or a CMOS (complementary metal-oxide semiconductor) image sensor is used. The analog signal output from the imaging device 36 is converted to a digital signal by the analog-digital conversion unit 38 and input to the processor device 16.

As illustrated in Fig. 2, the processor device 16 includes an image capture control unit 40, an image processing unit 42, an image obtaining unit 44, an image recognition unit 46, a notification control unit 58, an insertion-withdrawal determination unit 68, a display control unit 70, a storage control unit 72, and a storage unit 74.

The image capture control unit 40 controls the light source control unit 24 of the light source device 14, the imaging device 36 and the analog-digital conversion unit 38 of the endoscope 12, and the image processing unit 42 of the processor device 16 to thereby centrally control capturing of moving images and still images by the endoscope system 10.

The image processing unit 42 performs image processing for a digital signal input from the analog-digital conversion unit 38 of the endoscope 12 and generates image data (hereinafter expressed as an image) that represents an endoscopic image. The image processing unit 42 performs image processing that corresponds to the wavelength pattern of irradiation light at the time of image capturing.

The image obtaining unit 44 obtains an image generated by the image processing unit 42. The image obtaining unit 44 may obtain one image or a plurality of images. The image obtaining unit 44 may handle a moving image obtained by image capturing of a lumen of a subject in time series at a constant frame rate as a large number of consecutive images (still images). Note that the image obtaining unit 44 may obtain an image input by using the input unit 20 or an image stored in the storage unit 74. The image obtaining unit 44 may obtain an image from an external apparatus, such as a server, connected to a network not illustrated.

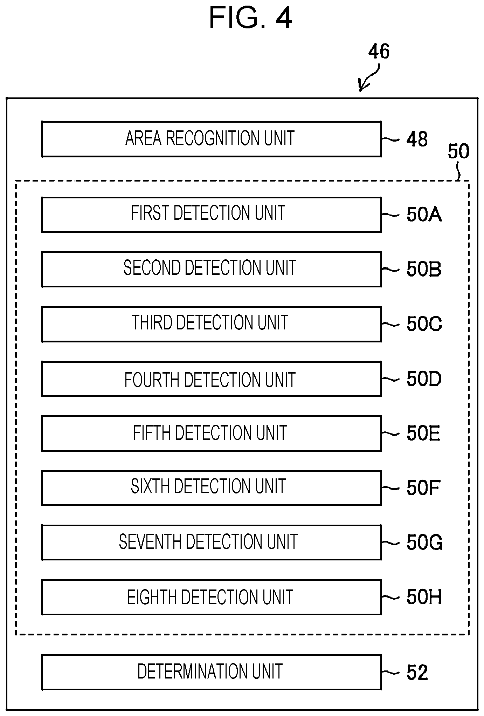

The image recognition unit 46 recognizes an image obtained by the image obtaining unit 44. Fig. 4 is a block diagram illustrating the configuration of the image recognition unit 46. As illustrated in Fig. 4, the image recognition unit 46 includes an area recognition unit 48, a detection unit 50, and a determination unit 52.

The area recognition unit 48 recognizes, from an image obtained by the image obtaining unit 44, an area (position) in the lumen in which the tip part 12D of the endoscope 12 is present. The area recognition unit 48 recognizes, for example, the rectum, the sigmoid colon, the descending colon, the transverse colon, the ascending colon, the cecum, the ileum, or the jejunum as the area in the lumen.

The area recognition unit 48 is a trained model trained by deep learning using a convolutional neural network. The area recognition unit 48 can recognize the area from the image by learning of, for example, images of mucous membranes of the respective areas.

Note that the area recognition unit 48 may obtain form information about the bending part 12C of the endoscope 12 by using an endoscope insertion-form observation apparatus (not illustrated) that includes, for example, a magnetic coil, and estimate the position of the tip part 12D from the form information. The area recognition unit 48 may obtain form information about the bending part 12C of the endoscope 12 by emitting an X ray from outside the subject and estimate the position of the tip part 12D from the form information.

The detection unit 50 (an example of a region-of-interest detection unit) detects a lesion that is a region of interest from an input image and recognizes the position of the lesion in the image. The lesion described here is not limited to a lesion caused by a disease and includes a region that is in a state different from a normal state in appearance. Examples of the lesion include a polyp, cancer, a colon diverticulum, inflammation, a scar from treatment, such as an EMR (endoscopic mucosal resection) scar or an ESD (endoscopic submucosal dissection) scar, a clipped part, a bleeding point, perforation, and an atypical vessel.

The detection unit 50 includes a first detection unit 50A, a second detection unit 50B, a third detection unit 50C, a fourth detection unit 50D, a fifth detection unit 50E, a sixth detection unit 50F, a seventh detection unit 50G, and an eighth detection unit 50H that correspond to the respective areas in the lumen. Here, for example, the first detection unit 50A corresponds to the rectum, the second detection unit 50B corresponds to the sigmoid colon, the third detection unit 50C corresponds to the descending colon, the fourth detection unit 50D corresponds to the transverse colon, the fifth detection unit 50E corresponds to the ascending colon, the sixth detection unit 50F corresponds to the cecum, the seventh detection unit 50G corresponds to the ileum, and the eighth detection unit 50H corresponds to the jejunum.

The first detection unit 50A, the second detection unit 50B, the third detection unit 50C, the fourth detection unit 50D, the fifth detection unit 50E, the sixth detection unit 50F, the seventh detection unit 50G, and the eighth detection unit 50H are trained models. These trained models are models trained by using different datasets. More specifically, the plurality of trained models are models trained by using respective datasets formed of images obtained by image capturing of different areas in the lumen.

That is, the first detection unit 50A is a model trained by using a dataset formed of images of the rectum, the second detection unit 50B is a model trained by using a dataset formed of images of the sigmoid colon, the third detection unit 50C is a model trained by using a dataset formed of images of the descending colon, the fourth detection unit 50D is a model trained by using a dataset formed of images of the transverse colon, the fifth detection unit 50E is a model trained by using a dataset formed of images of the ascending colon, the sixth detection unit 50F is a model trained by using a dataset formed of images of the cecum, the seventh detection unit 50G is a model trained by using a dataset formed of images of the ileum, and the eighth detection unit 50H is a model trained by using a dataset formed of images of the jejunum.

It is desirable to obtain these trained models by deep learning using a convolutional neural network. Alternatively, a support vector machine may be used.

The detection unit 50 detects a lesion by using a detection unit corresponding to the area in the lumen recognized by the area recognition unit 48 among the first detection unit 50A, the second detection unit 50B, the third detection unit 50C, the fourth detection unit 50D, the fifth detection unit 50E, the sixth detection unit 50F, the seventh detection unit 50G, and the eighth detection unit 50H.

That is, the detection unit 50 detects a lesion by using the first detection unit 50A when the area in the lumen is the rectum, using the second detection unit 50B when the area in the lumen is the sigmoid colon, using the third detection unit 50C when the area in the lumen is the descending colon, using the fourth detection unit 50D when the area in the lumen is the transverse colon, using the fifth detection unit 50E when the area in the lumen is the ascending colon, using the sixth detection unit 50F when the area in the lumen is the cecum, using the seventh detection unit 50G when the area in the lumen is the ileum, or using the eighth detection unit 50H when the area in the lumen is the jejunum.

The first detection unit 50A, the second detection unit 50B, the third detection unit 50C, the fourth detection unit 50D, the fifth detection unit 50E, the sixth detection unit 50F, the seventh detection unit 50G, and the eighth detection unit 50H are trained for the respective areas in the lumen, which enables appropriate detection of the respective areas.

The determination unit 52 determines whether a lesion detected by the detection unit 50 is benign or malignant. The determination unit 52 is a trained model trained by deep learning using a convolutional neural network. As in the detection unit 50, the determination unit 52 may be formed of identification units for the respective areas.

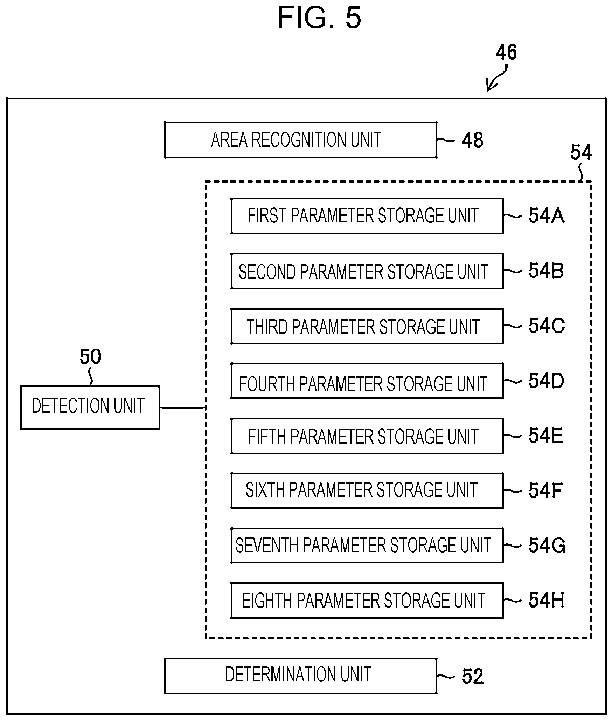

Fig. 5 is a block diagram illustrating another form of the configuration of the image recognition unit 46. As illustrated in Fig. 5, the image recognition unit 46 of this form includes one detection unit 50 and a parameter storage unit 54. The parameter storage unit 54 includes a first parameter storage unit 54A, a second parameter storage unit 54B, a third parameter storage unit 54C, a fourth parameter storage unit 54D, a fifth parameter storage unit 54E, a sixth parameter storage unit 54F, a seventh parameter storage unit 54G, and an eighth parameter storage unit 54H that store parameters for detecting the respective areas in the lumen. Here, for example, the first parameter storage unit 54A stores a parameter for detecting the rectum, the second parameter storage unit 54B stores a parameter for detecting the sigmoid colon, the third parameter storage unit 54C stores a parameter for detecting the descending colon, the fourth parameter storage unit 54D stores a parameter for detecting the transverse colon, the fifth parameter storage unit 54E stores a parameter for detecting the ascending colon, the sixth parameter storage unit 54F stores a parameter for detecting the cecum, the seventh parameter storage unit 54G stores a parameter for detecting the ileum, and the eighth parameter storage unit 54H stores a parameter for detecting the jejunum.

The parameters are parameters of trained models. The plurality of trained models are models trained by using different datasets.

The detection unit 50 detects a lesion by using a parameter corresponding to the area in the lumen recognized by the area recognition unit 48 among the parameters stored in the first parameter storage unit 54A, the second parameter storage unit 54B, the third parameter storage unit 54C, the fourth parameter storage unit 54D, the fifth parameter storage unit 54E, the sixth parameter storage unit 54F, the seventh parameter storage unit 54G, and the eighth parameter storage unit 54H.

Specifically, the detection unit 50 detects a lesion by using the parameter stored in the first parameter storage unit 54A when the area in the lumen is the rectum, using the parameter stored in the second parameter storage unit 54B when the area in the lumen is the sigmoid colon, using the parameter stored in the third parameter storage unit 54C when the area in the lumen is the descending colon, using the parameter stored in the fourth parameter storage unit 54D when the area in the lumen is the transverse colon, using the parameter stored in the fifth parameter storage unit 54E when the area in the lumen is the ascending colon, using the parameter stored in the sixth parameter storage unit 54F when the area in the lumen is the cecum, using the parameter stored in the seventh parameter storage unit 54G when the area in the lumen is the ileum, or using the parameter stored in the eighth parameter storage unit 54H when the area in the lumen is the jejunum.

The first parameter storage unit 54A, the second parameter storage unit 54B, the third parameter storage unit 54C, the fourth parameter storage unit 54D, the fifth parameter storage unit 54E, the sixth parameter storage unit 54F, the seventh parameter storage unit 54G, and the eighth parameter storage unit 54H store the parameters trained for the respective areas in the lumen, which enables appropriate detection of the respective areas.

Referring back to Fig. 2, the recognition result notification unit 60 is connected to the notification control unit 58. The recognition result notification unit 60 (an example of a detection result notification unit) is notification means for giving a notification of the result of image recognition by the image recognition unit 46. The notification control unit 58 controls the recognition result notification unit 60 to give a notification.

The insertion-withdrawal determination unit 68 determines whether the step in the examination is the insertion step or the withdrawal step.

The insertion-withdrawal determination unit 68 detects a motion vector from a plurality of images generated by the image processing unit 42, determines the movement direction of the insertion part 12A on the basis of the detected motion vector, and determines the step in the examination from the movement direction. To detect the motion vector, a publicly known method, such as a block matching algorithm, can be used.

Note that the insertion-withdrawal determination unit 68 may determine the movement direction of the insertion part 12A from information from a sensor (not illustrated) provided in the insertion part 12A and determine the step in the examination from the movement direction. The insertion-withdrawal determination unit 68 may determine the movement direction of the insertion part 12A by using an endoscope insertion-form observation apparatus (not illustrated) that includes, for example, a magnetic coil, and determine the step in the examination from the movement direction.

The insertion-withdrawal determination unit 68 may determine that a step from when the examination starts to when the insertion part 12A reaches the return point is the insertion step and a step after the insertion part 12A has reached the return point is the withdrawal step. The insertion-withdrawal determination unit 68 may determine whether the insertion part 12A reaches the return point on the basis of input from the input unit 20 by the doctor.

The insertion-withdrawal determination unit 68 may automatically recognize that the insertion part 12A reaches the return point. For example, the insertion-withdrawal determination unit 68 may perform automatic recognition from an image generated by the image processing unit 42 or perform automatic recognition using an endoscope insertion-form observation apparatus not illustrated. In a case where the insertion-withdrawal determination unit 68 performs automatic recognition, the return point can be, for example, the Bauhin's valve in a case of a lower gastrointestinal endoscope or can be, for example, the duodenum in a case of an upper gastrointestinal endoscope.

The insertion-withdrawal determination unit 68 may recognize that the insertion part 12A reaches the return point from an operation of the endoscope 12 by the doctor. For example, a point at which a flipping operation is performed may be determined to be the return point in a case of an upper gastrointestinal endoscope. In a case where the insertion-withdrawal determination unit 68 performs automatic recognition, some of these automatic recognition methods may be combined.

The display control unit 70 displays an image generated by the image processing unit 42 on the display unit 18.

The storage control unit 72 stores an image generated by the image processing unit 42 in the storage unit 74. For example, the storage control unit 72 stores in the storage unit 74, for example, an image captured in accordance with an instruction for obtaining a still image and information about the wavelength pattern of the irradiation light L0 used at the time of image capturing.

The storage unit 74 is, for example, a storage device, such as a hard disk. Note that the storage unit 74 is not limited to a device built in the processor device 16. For example, the storage unit 74 may be an external storage device (not illustrated) connected to the processor device 16. The external storage device may be connected to the processor device 16 via a network not illustrated.

The endoscope system 10 thus configured captures a moving image or a still image and displays the captured image on the display unit 18. The endoscope system 10 performs image recognition for the captured image, and the recognition result notification unit 60 gives a notification of the results of recognition.

### Method for Notification of Recognition Results

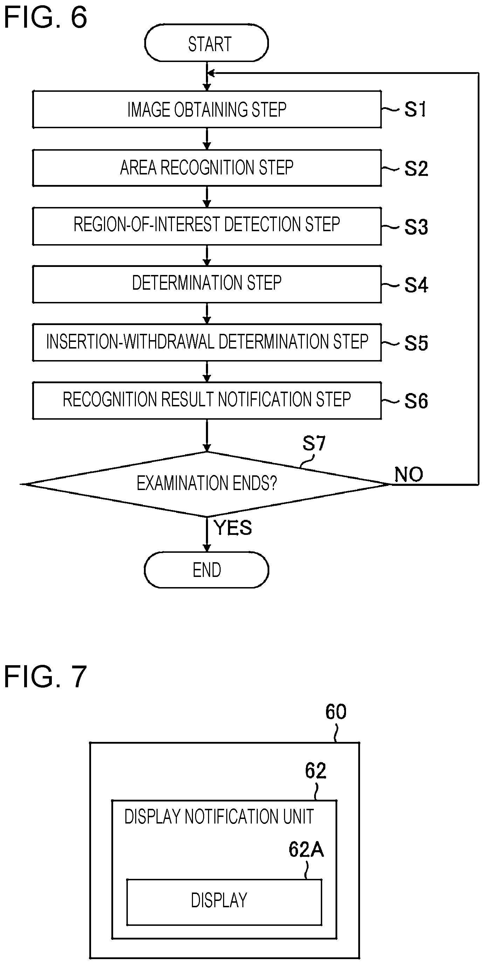

Fig. 6 is a flowchart illustrating processes of a method for notification of recognition results by the endoscope system 10. The method for notification of recognition results has an image obtaining step (step S1), an area recognition step (step S2), a region-of-interest detection step (step S3), a determination step (step S4), an insertion-withdrawal determination step (step S5), a recognition result notification step (step S6), and an end determination step (step S7).

### Image Obtaining Step (Step S1)

In step S1, the endoscope system 10 captures a moving image at a constant frame rate in accordance with control by the image capture control unit 40.

That is, the light source control unit 24 sets the ratio between the amount of light emitted from the first laser light source 22A and the amount of light emitted from the second laser light source 22B so as to correspond to a desired observation mode. Accordingly, the observation target region in the lumen of the subject is irradiated with the irradiation light L0 having a desired wavelength pattern.

The image capture control unit 40 controls the imaging device 36, the analog-digital conversion unit 38, and the image processing unit 42 to capture an image of the observation target region by receiving reflected light from the observation target region. The display control unit 70 displays the captured image on the display unit 18. The image obtaining unit 44 obtains the captured image.

### Area Recognition Step (Step S2)

Next, in step S2, the area recognition unit 48 recognizes from the image obtained by the image obtaining unit 44, an area in the lumen in which the tip part 12D is present. Here, the area recognition unit 48 recognizes the area as one of the rectum, the sigmoid colon, the descending colon, the transverse colon, the ascending colon, the cecum, the ileum, or the jejunum.

### Region-of-Interest Detection Step (Step S3)

In step S3, the detection unit 50 detects a lesion from the image obtained by the image obtaining unit 44 on the basis of the area recognized by the area recognition unit 48. Determination Step (Step S4)

In step S4, the determination unit 52 determines whether the lesion detected in step S3 is benign or malignant.

### Insertion-Withdrawal Determination Step (Step S5)

In step S5, the insertion-withdrawal determination unit 68 detects a motion vector from a plurality of time-series images generated by the image processing unit 42, detects the movement direction of the insertion part 12A on the basis of the detected motion vector, and determines whether the step in the examination is the insertion step or the withdrawal step from the movement direction.

### Recognition Result Notification Step (Step S6)

In step S6, the notification control unit 58 causes the recognition result notification unit 60 to give a notification of the results of recognition in step S2, step S3, and step S4. In a case where the step in the examination is the withdrawal step, the notification control unit 58 causes the recognition result notification unit 60 to give a notification that is more noticeable than in a case where the step in the examination is the insertion step.

That is, in the case where the step in the examination is the insertion step, it is assumed that the doctor is washing the lumen. Therefore, in the case where the step in the examination is the insertion step, it is desirable to give a notification that is less noticeable so as not to hinder the doctor's operation. On the other hand, in a case where the step in the examination is the withdrawal step, it is assumed that the doctor is observing the lumen. Therefore, to appropriately support the diagnosis, a notification that is more noticeable is given. Accordingly, in step S6, it is possible to call the doctor's attention with more certainty in the withdrawal step.

### End Determination Step (Step S7)

In step S7, it is determined whether the examination using the endoscope system 10 ends. In a case where the examination does not end, the flow returns to step S1, and the same processes are repeated. In a case where the examination ends, the processes in the flowchart end.

Accordingly, it is determined whether the step in the examination is the insertion step or the withdrawal step, and the recognition result notification unit 60 is caused to give a notification in accordance with the determined step. Therefore, a notification of the results of recognition can be appropriately given, and a notification of the result of detection of the lesion that is the region of interest can be appropriately given.

### First Embodiment

Fig. 7 is a block diagram illustrating the configuration of the recognition result notification unit 60 according to a first embodiment. In this embodiment, the recognition result notification unit 60 includes a display notification unit 62. Further, the display notification unit 62 includes a display 62A. The display 62A is a display device that outputs and displays information, such as an image. When a lesion is detected, the display notification unit 62 gives a notification of detection of the lesion by display on the display 62A. In this embodiment, the display 62A and the display unit 18 are implemented as a common display.

In the first embodiment, in a case where the step in the examination is the withdrawal step, the notification control unit 58 causes the display notification unit 62 to give a notification of detection of a lesion such that the notification is more noticeable than in the case of the insertion step.

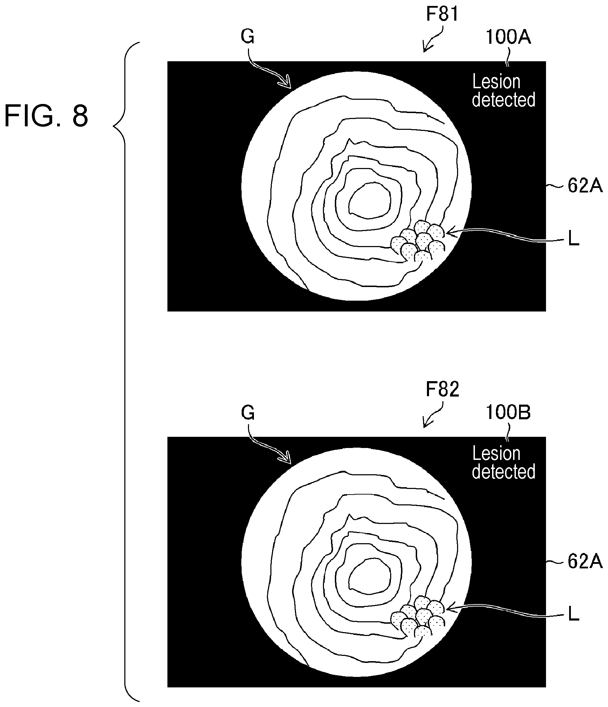

Fig. 8 includes diagrams illustrating examples where a notification of detection of a lesion from an image is given by displaying text. In the examples illustrated in Fig. 8, on the display 62A that is displaying an image G generated by the image processing unit 42, the notification control unit 58 controls the display notification unit 62 to give a notification that a lesion L has been detected from the image G, by displaying text. In Fig. 8, F81 illustrates a case where the step in the examination using the insertion part 12A is the insertion step. In the case of F81, text 100A having a first size is displayed on the display 62A. In Fig. 8, F82 illustrates a case where the step to be performed in the examination using insertion part 12A is the withdrawal step. In the case of F82, text 100B having a second size larger than the first size is displayed on the display 62A.

Accordingly, in the examples illustrated in Fig. 8, the text size is made different to thereby make the notification in the case of the withdrawal step more noticeable than in the case of the insertion step. Note that the text size need not be made different, and the text color may be changed. In this case, a notification using text in a color that is relatively high in brightness or saturation is more noticeable than a notification using text in a color that is relatively low in brightness or saturation. A notification using text having a red hue is more noticeable than a notification using text having a blue hue.

To give a notification of detection of a lesion by display on the display 62A, the form in which the noticeability is made different depending on the step in the examination is not limited to the example form in which the text size or color is changed, and various forms are possible.

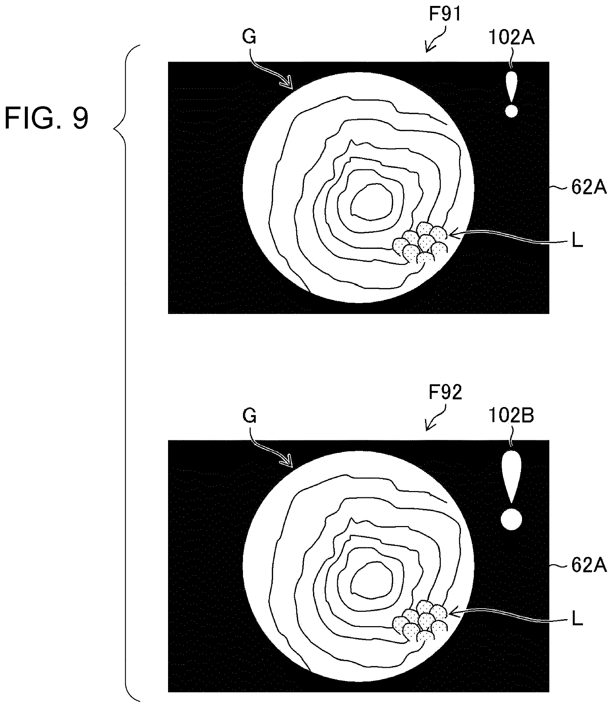

Fig. 9 includes diagrams illustrating examples where a notification of detection of a lesion from an image is given by displaying an icon. In the examples illustrated in Fig. 9, on the display 62A that is displaying the image G generated by the image processing unit 42, the notification control unit 58 controls the display notification unit 62 to give a notification that the lesion L has been detected from the image G, by displaying an icon. In Fig. 9, F91 illustrates a case where the step in the examination is the insertion step. In the case of F91, an icon 102A having the first size is displayed on the display 62A. In Fig. 9, F92 illustrates a case where the step in the examination is the withdrawal step. In the case of F92, an icon 102B having the second size larger than the first size is displayed on the display 62A.

Accordingly, when the icon size is made different, a notification in the case of the withdrawal step can be made more noticeable than in the case of the insertion step.

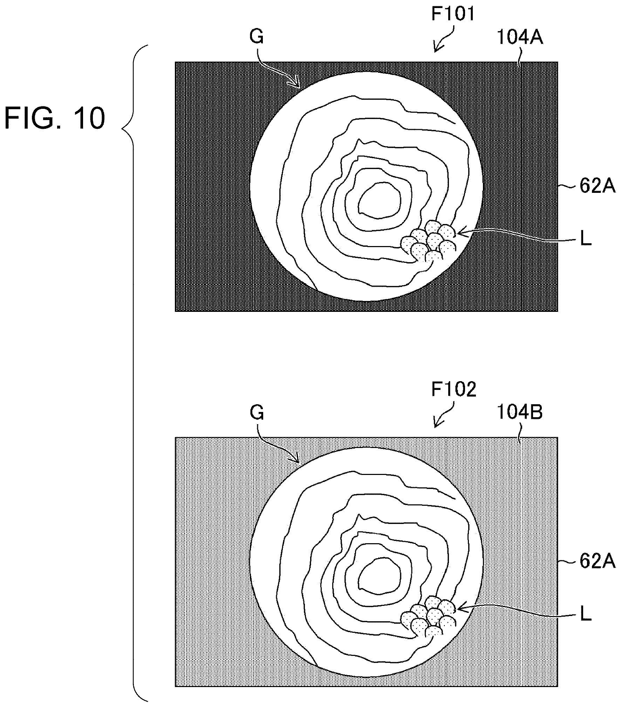

Fig. 10 includes diagrams illustrating examples where a notification of detection of a lesion from an image is given by displaying a background. In the examples illustrated in Fig. 10, on the display 62A that is displaying the image G generated by the image processing unit 42, the notification control unit 58 controls the display notification unit 62 to give a notification that the lesion L has been detected from the image G, by displaying a background. The background is a region other than the image G in the display area of the display 62A. Usually, a black background is displayed.

In Fig. 10, F101 illustrates a case where the step in the examination is the insertion step. In the case of F101, a background 104A in a first background color different from black is displayed on the display 62A. In Fig. 10, F102 illustrates a case where the step in the examination is the withdrawal step. In the case of F102, a background 104B in a second background color higher in brightness than the first background color is displayed on the display 62A.

Accordingly, when the brightness of the background color is made different, a notification in the case of the withdrawal step can be made more noticeable than in the case of the insertion step. Note that the brightness of the background color need not be made different, and the saturation or hue of the background color may be changed. In this case, a notification using a background color that is higher in saturation is more noticeable. A notification using a background color of a red hue is more noticeable than a notification using a background color of a blue hue.

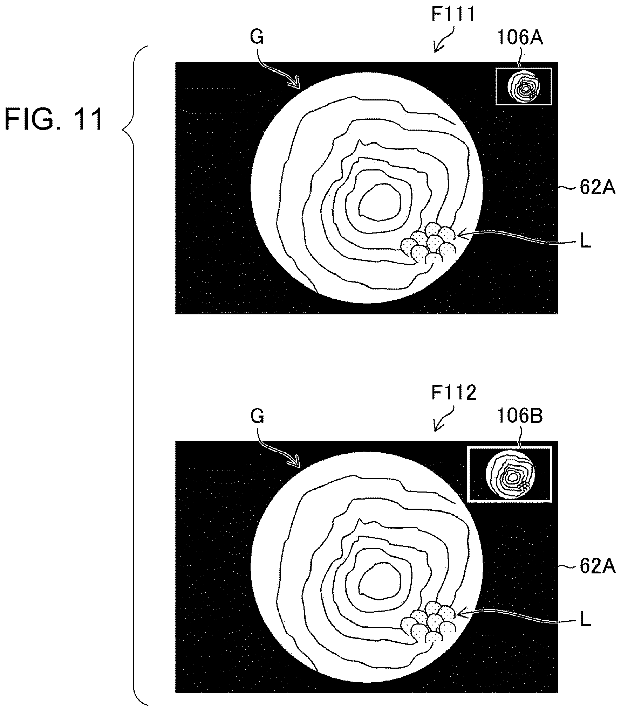

Fig. 11 includes diagrams illustrating examples where a notification of detection of a lesion from an image is given by displaying a frame. In the examples illustrated in Fig. 11, on the display 62A that is displaying the image G generated by the image processing unit 42, the notification control unit 58 controls the display notification unit 62 to give a notification that the lesion L has been detected from the image G, by displaying a frame in which the lesion L is included. In Fig. 11, F111 illustrates a case where the step in the examination is the insertion step. In the case of F111, a frame 106A having the first size is displayed on the display 62A together with the image G. In the frame 106A, the image G displayed on the display 62A is displayed. The notification control unit 58 may display in the frame 106A, the image previous to the image from which the lesion L has been detected, instead of the image G displayed on the display 62A.

In Fig. 11, F112 illustrates a case where the step in the examination is the withdrawal step. In the case of F112, a frame 106B having the second size larger than the first size is displayed on the display 62A together with the image G. The image displayed in the frame 106B needs to be the image as in the case of F 111.

Accordingly, when the size of the frame in which the image G including the lesion L is displayed is made different, a notification in the case of the withdrawal step can be made more noticeable than in the case of the insertion step.

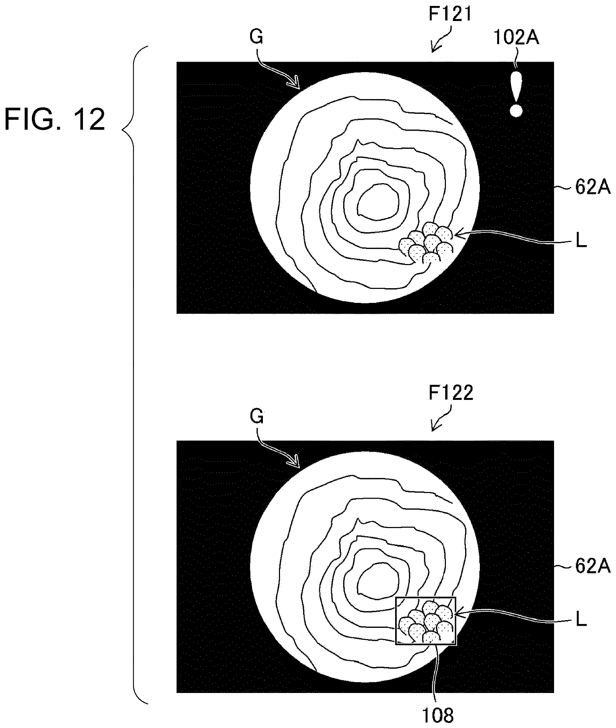

Fig. 12 includes diagrams illustrating examples where a notification of detection of a lesion from an image is given by display in different forms. In the examples illustrated in Fig. 12, on the display 62A that is displaying the image G generated by the image processing unit 42, the notification control unit 58 controls the display notification unit 62 to give a notification that the lesion L has been detected from the image G, in a display form that differs depending on the step determined by the insertion-withdrawal determination unit 68. In Fig. 12, F121 illustrates a case where the step in the examination is the insertion step. In the case of F121, the icon 102A having the first size is displayed on the display 62A. The icon 102A may be hidden. In Fig. 12, F122 illustrates a case where the step in the examination is the withdrawal step. In the case of F122, a geometric shape 108 that indicates the area of the lesion L is superimposed on the image G and displayed at the position of the lesion L on the display 62A.

Accordingly, when the form of display for notifying that the lesion L has been detected is made different depending on the step determined by the insertion-withdrawal determination unit 68, a notification in the case of the withdrawal step can be made more noticeable than in the case of the insertion step.

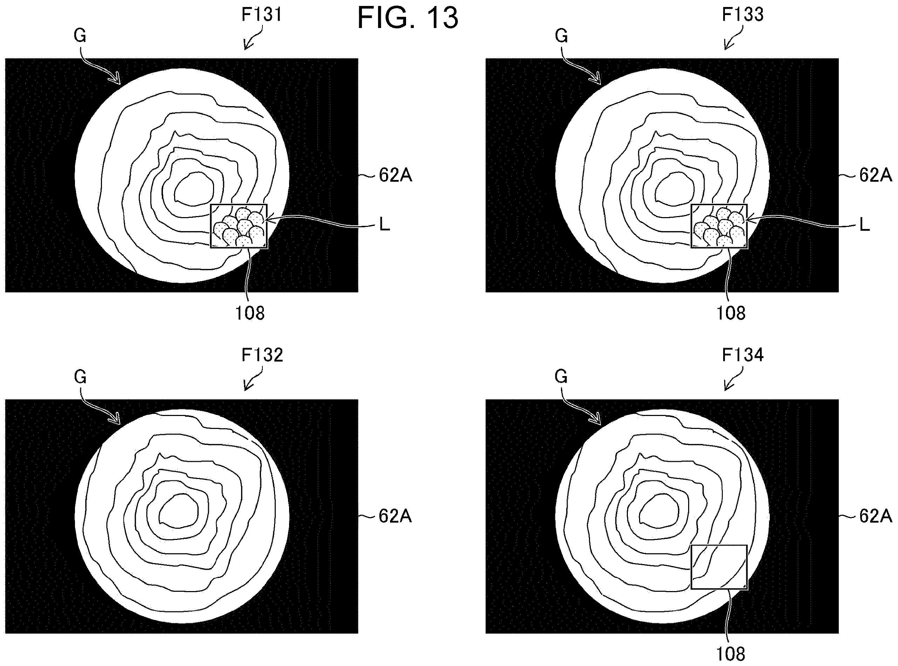

Fig. 13 includes diagrams illustrating examples where a notification of detection of a lesion from an image is given by display for different display periods. In the examples illustrated in Fig. 13, on the display 62A that is displaying the image G generated by the image processing unit 42, the notification control unit 58 controls the display notification unit 62 to give a notification that the lesion L has been detected from the image G, by display for different display periods. In Fig. 13, F131 and F132 illustrate a case where the step in the examination is the insertion step. In the case of F131, the geometric shape 108 that indicates the area of the lesion L is superimposed on the image G and displayed at the position of the lesion L on the display 62A. F132 illustrates a case where a certain time has elapsed since F131 and the insertion part 12A has moved further in the insertion direction. In the case of F132, the lesion L is not detected from the image G, and the geometric shape 108 is not displayed. That is, in a case where the step in the examination is the insertion step, only at the time of detection of the lesion L, the notification control unit 58 superimposes on the image G and displays the geometric shape 108 that indicates the area of the lesion at the position of the lesion.

On the other hand, in Fig. 13, F133 and F134 illustrate a case where the step in the examination is the withdrawal step. In the case of F133, the geometric shape 108 that indicates the area of the lesion L is superimposed on the image G and displayed at the position of the lesion L on the display 62A. F 134 illustrates a case where a certain time has elapsed since F133 and the insertion part 12A has moved further in the withdrawal direction. In the case of F134, the lesion L is not detected from the image G, but the geometric shape 108 displayed in F133 is kept displayed at the same position. That is, in a case where the step in the examination is the withdrawal step, the notification control unit 58 superimposes on the image G and displays the geometric shape 108 that indicates the area of the lesion at the position of the lesion at the time of detection of the lesion L and keeps displaying the geometric shape 108 for a certain period after the detection.

Accordingly, when the display period of the geometric shape that indicates the area of the lesion L is made different, a notification in the case of the withdrawal step can be made more noticeable than in the case of the insertion step.

In this embodiment, it is assumed that the display unit 18 and the display 62A are implemented as a common display; however, the image G may be displayed on the display unit 18, and a notification of the result of detection of a lesion may be displayed on the display 62A.

### Second Embodiment

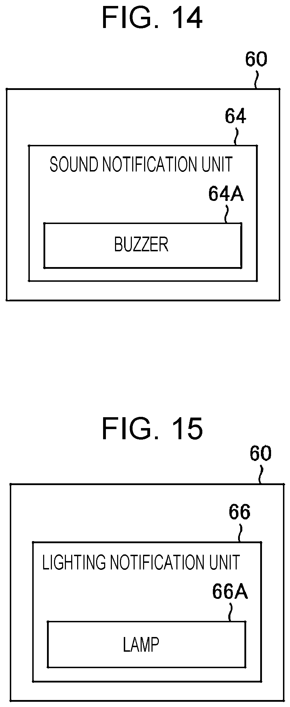

Fig. 14 is a block diagram illustrating the configuration of the recognition result notification unit 60 according to a second embodiment. In this embodiment, the recognition result notification unit 60 includes a sound notification unit 64. Further, the sound notification unit 64 includes a buzzer 64A. The buzzer 64A is a sound generation device that generates a notification sound and, for example, a piezoelectric buzzer having a piezoelectric element is used. When a lesion is detected, the sound notification unit 64 gives a notification of detection of the lesion by a notification sound from the buzzer 64A. In this embodiment, the buzzer 64A is provided in the processor device 16.

In the second embodiment, in a case where the step in the examination is the withdrawal step, the notification control unit 58 causes the sound notification unit 64 to give a notification of detection of a lesion such that the notification is more noticeable than in the case of the insertion step. That is, the notification control unit 58 causes the sound notification unit 64 to output a sound at a first sound volume (loudness of sound) in the insertion step and causes the sound notification unit 64 to output a sound at a second sound volume higher than the first sound volume in the withdrawal step.

Accordingly, in the second embodiment, the sound volume is made different to thereby make a notification in the case of the withdrawal step more noticeable than in the case of the insertion step. The sound volume need not be made different, and the sound length may be changed. In this case, a relatively long sound is more noticeable than a relatively short sound.

### Third Embodiment

Fig. 15 is a block diagram illustrating the configuration of the recognition result notification unit 60 according to a third embodiment. In this embodiment, the recognition result notification unit 60 includes a lighting notification unit 66. Further, the lighting notification unit 66 includes a lamp 66A. The lamp 66A is a light source that generates notification light and, for example, a light emitting diode is used. When a lesion is detected, the lighting notification unit 66 gives a notification of detection of the lesion by lighting the lamp 66A. In this embodiment, the lamp 66A is provided in the processor device 16.

In the third embodiment, in a case where the step in the examination is the withdrawal step, the notification control unit 58 causes the lighting notification unit 66 to give a notification of detection of a lesion such that the notification is more noticeable than in the case of the insertion step. That is, the notification control unit 58 causes the lighting notification unit 66 to light the lamp 66A with a first amount of light (intensity of light) in the insertion step and causes the lighting notification unit 66 to output the lamp 66A with a second amount of light larger than the first amount of light in the withdrawal step.

Accordingly, in the third embodiment, the amount of light is made different to thereby make a notification in the case of the withdrawal step more noticeable than in the case of the insertion step. The amount of light need not be made different, and the color of light may be changed. For example, red-hue lighting is more noticeable than blue-hue lighting. Further the duration of lighting may be made different. For example, a continuous lighting state where the duration of lighting is relatively long is more noticeable than a blinking state where the duration of lighting is relatively short.

### Fourth Embodiment

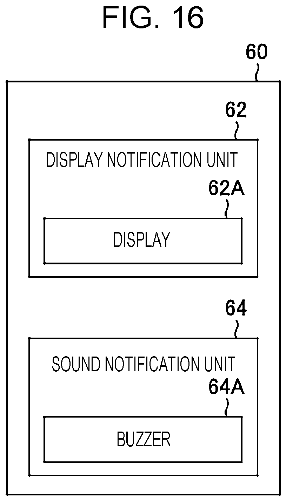

Fig. 16 is a block diagram illustrating the configuration of the recognition result notification unit 60 according to a fourth embodiment. In this embodiment, the recognition result notification unit 60 includes the display notification unit 62 and the sound notification unit 64.

In the fourth embodiment, in a case where the step in the examination is the withdrawal step, the notification control unit 58 causes the recognition result notification unit 60 to give a notification of detection of a lesion such that the notification is more noticeable than in the case of the insertion step. Specifically, in the insertion step, the notification control unit 58 causes the display notification unit 62 to superimpose on the image G and display the geometric shape 108 that indicates the area of the lesion L at the position of the lesion L on the display 62A as in F122 in Fig. 12. In the withdrawal step, the notification control unit 58 causes the display notification unit 62 to superimpose on the image G and display the geometric shape 108 that indicates the area of the lesion L at the position of the lesion L on the display 62A as in the insertion step and causes the sound notification unit 64 to output a sound.

Accordingly, in the fourth embodiment, display is similarly performed on the display 62A, and output of a sound from the buzzer 64A is made different to thereby make a notification in the case of the withdrawal step more noticeable than in the case of the insertion step.

### Fifth Embodiment

The configuration of the recognition result notification unit 60 according to a fifth embodiment is the same as the configuration of the recognition result notification unit 60 according to the fourth embodiment.

In the fifth embodiment, in a case where the step in the examination is the withdrawal step, the notification control unit 58 causes the recognition result notification unit 60 to give a notification of detection of a lesion such that the notification is more noticeable than in the case of the insertion step. Specifically, in the insertion step, the notification control unit 58 causes the sound notification unit 64 to output a sound. In the withdrawal step, the notification control unit 58 causes the sound notification unit 64 to output a sound and causes the display notification unit 62 to display the icon 102B on the display 62A as in F92 in Fig. 9.

Accordingly, in the fifth embodiment, a sound is similarly output from the buzzer 64A, and display on the display 62A is made different to thereby make a notification in the case of the withdrawal step more noticeable than in the case of the insertion step.

### Sixth Embodiment

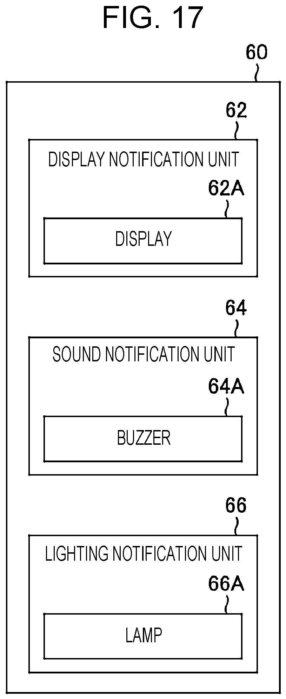

Fig. 17 is a block diagram illustrating the configuration of the recognition result notification unit 60 according to a sixth embodiment. In this embodiment, the recognition result notification unit 60 includes the display notification unit 62, the sound notification unit 64, and the lighting notification unit 66.

In the sixth embodiment, in a case where the step in the examination is the withdrawal step, the notification control unit 58 causes the recognition result notification unit 60 to give a notification of detection of a lesion such that the notification is more noticeable than in the case of the insertion step. Specifically, in the insertion step, the notification control unit 58 causes the lighting notification unit 66 to light the lamp 66A. In the withdrawal step, the notification control unit 58 causes the lighting notification unit 66 to light the lamp 66A, causes the display notification unit 62 to superimpose on the image G and display the geometric shape 108 that indicates the area of the lesion L at the position of the lesion L on the display 62A, and causes the sound notification unit 64 to output a sound from the buzzer 64A.

Accordingly, in the sixth embodiment, the lamp 66A is similarly lit, and display on the display 62A and output of a sound from the buzzer 64A are made different to thereby make a notification in the case of the withdrawal step more noticeable than in the case of the insertion step.

When N is an integer from 0 to 2, the notification control unit 58 may cause N units among the display notification unit 62, the sound notification unit 64, and the lighting notification unit 66 to give a notification in the insertion step and may cause at least (N+1) units among the display notification unit 62, the sound notification unit 64, and the lighting notification unit 66 to give a notification in the withdrawal step. As the notification given by the display notification unit 62, any of the notification by displaying text, the notification by displaying an icon, the notification by changing the background color, the notification by displaying a frame, or the notification by displaying a geometric shape that indicates the area of a lesion may be used. The notification by the sound notification unit 64 is a notification by a notification sound from the buzzer 64A. The notification by the lighting notification unit 66 is a notification by lighting the lamp 66A.

When notifications are thus given, a notification in the case of the withdrawal step is made more noticeable than in the case of the insertion step. The method for notification may be any method as long as the notification is made more noticeable in the case of the withdrawal step than in the case of the insertion step such that the method is expected to call the doctor's attention with more certainty.

In the embodiments described above, the hardware configuration of the processing units that perform various types of processing of, for example, the image recognition unit 46, the notification control unit 58, and the insertion-withdrawal determination unit 68 is implemented as various processors as described below. The various processors include a CPU (central processing unit), which is a general-purpose processor executing software (program) to function as various processing units, a GPU (graphics processing unit), which is a processor specialized in image processing, a programmable logic device (PLD), such as an FPGA (field-programmable gate array), which is a processor having a circuit configuration that is changeable after manufacture, and a dedicated electric circuit, such as an ASIC (application-specific integrated circuit), which is a processor having a circuit configuration specifically designed to perform specific processing.

One processing unit may be configured as one of the various processors or two or more processors of the same type or different types (for example, a plurality of FPGAs, a combination of a CPU and an FPGA, or a combination of a CPU and a GPU). Further, a plurality of processing units may be configured as one processor. As the first example of configuring a plurality of processing units as one processor, a form is possible where one or more CPUs and software are combined to configure one processor, and the processor functions as the plurality of processing units, a representative example of which is a computer, such as a client or a server. As the second example thereof, a form is possible where a processor is used in which the functions of the entire system including the plurality of processing units are implemented as one IC (integrated circuit) chip, a representative example of which is a system on chip (SoC). As described above, regarding the hardware configuration, the various processing units are configured by using one or more of the various processors described above.

Further, the hardware configuration of the various processors is more specifically an electric circuit (circuitry) in which circuit elements, such as semiconductor elements, are combined.

### Reference Signs List

- 10: endoscope system
- 12: endoscope
- 12A: insertion part
- 12B: operation part
- 12C: bending part
- 12D: tip part
- 12E: angle knob
- 13: mode switching switch
- 14: light source device
- 16: processor device
- 18: display unit
- 20: input unit
- 22A: first laser light source
- 22B: second laser light source
- 24: light source control unit
- 28A: optical fiber
- 28B: optical fiber
- 30: fluorescent body
- 32: diffusion member
- 34: image capturing lens
- 36: imaging device
- 38: analog-digital conversion unit
- 40: image capture control unit
- 42: image processing unit
- 44: image obtaining unit
- 46: image recognition unit
- 48: area recognition unit
- 50: detection unit
- 50A: first detection unit
- 50B: second detection unit
- 50C: third detection unit
- 50D: fourth detection unit
- 50E: fifth detection unit
- 50F: sixth detection unit
- 50G: seventh detection unit
- 50H: eighth detection unit
- 52: determination unit
- 54: parameter storage unit
- 54A: first parameter storage unit
- 54B: second parameter storage unit
- 54C: third parameter storage unit
- 54D: fourth parameter storage unit
- 54E: fifth parameter storage unit
- 54F: sixth parameter storage unit
- 54G: seventh parameter storage unit
- 54H: eighth parameter storage unit
- 58: notification control unit
- 60: recognition result notification unit
- 62: display notification unit
- 62A: display
- 64: sound notification unit
- 64A: buzzer
- 66: lighting notification unit
- 66A: lamp
- 68: insertion-withdrawal determination unit
- 70: display control unit
- 72: storage control unit
- 74: storage unit
- 100A: text
- 100B: text
- 102A: icon
- 102B: icon
- 106A: frame
- 106B: frame
- 108: geometric shape
- G: image
- L: lesion
- L1: light
- L11: excitation light
- L12: laser light
- L2: light
- S1 to S7: processes of method for notification of recognition results

## Claims

1. An endoscope system (10) for performing an examination of a lumen of a patient, the endoscope system comprising:
an insertion part (12A) configured to be inserted into the lumen;
a camera configured to perform image capturing of the lumen to obtain an endoscopic image;
a region-of-interest detection unit (50) configured to detect a region of interest from the endoscopic image;
a detection result notification unit (60) configured to give a notification of a result of detection of the region of interest;
an insertion-withdrawal determination unit (68) configured to determine whether a step in the examination is an insertion step in which the insertion part is inserted up to a return point in the lumen or a withdrawal step in which the insertion part is withdrawn from the return point; and
a notification control unit (58) configured to cause the detection result notification unit to give the notification in accordance with the step determined by the insertion-withdrawal determination unit; wherein
the detection result notification unit (60) comprises a display notification unit (62) configured to give a notification of detection of the region of interest by display on a display (62A), and **characterized in that**
the notification control unit (58) is configured to cause the display notification unit (62) to superimpose on the endoscopic image and display a geometric shape (108) that indicates an area of the region of interest at a position of the region of interest only at a time of detection of the region of interest in the insertion step, and to cause the display notification unit (62) to superimpose on the endoscopic image and display the geometric shape (108) that indicates the area of the region of interest at the position of the region of interest at the time of detection of the region of interest and keep displaying the geometric shape for a certain period after the detection in the withdrawal step.

2. The endoscope system according to claim 1, wherein
the notification control unit (58) is configured to cause the display notification unit (62) to display text (100A) having a first size in the insertion step, and to cause the display notification unit to display text (100B) having a second size larger than the first size in the withdrawal step.

3. The endoscope system according to claim 1 or 2, wherein
the notification control unit (58) is configured to cause the display notification unit (60) to display an icon having a first size in the insertion step, and to cause the display notification unit to display an icon having a second size larger than the first size in the withdrawal step.

4. The endoscope system according to any one of claims 1 to 3, wherein
the notification control unit (58) is configured to cause the display notification unit (60) to display a background in a first background color in the insertion step, and to cause the display notification unit to display a background in a second background color higher in brightness than the first background color in the withdrawal step.

5. The endoscope system according to any one of claims 1 to 4, wherein
the notification control unit (58) is configured to cause the display notification unit (60) to display a frame (106A) having a first size and including the region of interest together with the endoscopic image in the insertion step, and to cause the display notification unit to display a frame (106B) having a second size larger than the first size and including the region of interest together with the endoscopic image in the withdrawal step.

6. The endoscope system according to any one of claims 1 to 5, wherein
the detection result notification unit (60) comprises a sound notification unit (64) configured to give a notification of detection of the region of interest by outputting a sound, and
the notification control unit (58) is configured to cause the sound notification unit (64) to output the sound at a first sound volume in the insertion step, and to cause the sound notification unit to output the sound at a second sound volume higher than the first sound volume in the withdrawal step.

7. The endoscope system according to any one of claims 1 to 6, wherein
the detection result notification unit (60) comprises a lighting notification unit (66) configured to give a notification of detection of the region of interest by lighting a lamp, and
the notification control unit (58) is configured to cause the lighting notification unit (66) to light the lamp with a first amount of light in the insertion step, and to cause the lighting notification unit to light the lamp with a second amount of light larger than the first amount of light in the withdrawal step.

8. The endoscope system according to claim 1, wherein
the detection result notification unit (60) comprises
a sound notification unit configured to give a notification by outputting a sound, and
the notification control unit (58) is configured to cause the sound notification unit to output the sound in the withdrawal step.

9. The endoscope system according to claim 1, wherein
the detection result notification unit (60) comprises
a sound notification unit (64) configured to give a notification by outputting a sound, and
the notification control unit (58) is configured to cause the sound notification unit (64) to output the sound in the insertion step, and causes the sound notification unit to output the sound and causes the display notification unit to display an icon in the withdrawal step.

10. The endoscope system according to claim 1, wherein
the detection result notification unit (60) comprises
a sound notification unit (64) configured to give a notification by outputting a sound, and
a lighting notification unit (66) configured to give a notification by lighting a lamp (66A), and
the notification control unit (58) is configured to cause the lighting notification unit (66) to light the lamp (66A) in the insertion step, and causes the lighting notification unit (66) to light the lamp (66A), and to cause the sound notification unit (64) to output the sound in the withdrawal step.

11. The endoscope system according to claim 1, wherein
the detection result notification unit (60) comprises
a sound notification unit (64) configured to give a notification by outputting a sound, and
a lighting notification unit (66) configured to give a notification by lighting a lamp (66A), and
when N is an integer from 0 to 2, the notification control unit is configured to cause N units among the display notification unit (62), the sound notification unit (64), and the lighting notification unit (66) to give a notification in the insertion step, and causes at least (N+1) units among the display notification unit, the sound notification unit, and the lighting notification unit to give a notification in the withdrawal step.

## Patentansprüche

1. Ein Endoskopsystem (10) zur Durchführung einer Untersuchung eines Lumens eines Patienten, wobei das Endoskopsystem umfasst:
ein Einführteil (12A) , das so konfiguriert ist, dass es in das Lumen eingeführt werden kann;
eine Kamera, die so konfiguriert ist, dass sie Bildaufnahme des Lumens vornimmt, um ein endoskopisches Bild zu erhalten;
eine Erkennungseinheit (50 ) für den interessierenden Bereich, die so konfiguriert ist, dass sie einen interessierenden Bereich aus dem endoskopischen Bild erkennt;
eine Detektionsergebnis-Benachrichtigungseinheit (60), die so konfiguriert ist, dass sie eine Benachrichtigung über ein Ergebnis der Detektion des interessierenden Bereichs gibt;
eine Einführungs-/Entnahmebestimmungseinheit (68), die so konfiguriert ist, dass sie bestimmt, ob ein Schritt bei der Untersuchung ein Einführungsschritt ist, bei dem das Einführungsteil bis zu einem Rückführungspunkt in das Lumen eingeführt wird, oder ein Entnahmeschritt, bei dem das Einführungsteil vom Rückführungspunkt zurückgezogen wird; und
eine Benachrichtigungssteuereinheit (58), die so konfiguriert ist, dass sie veranlasst, die Detektionsergebnis-Benachrichtigungseinheit zu veranlassen, die Benachrichtigung in Übereinstimmung mit dem Schritt zu geben, der durch die Einfügungs-Entnahme-Bestimmungseinheit bestimmt wird; wobei
die Detektionsergebnis-Benachrichtigungseinheit (60) eine Anzeige-Benachrichtigungseinheit (62) umfasst, die so konfiguriert ist, dass sie eine Benachrichtigung bzw. Meldung über die Detektion des interessierenden Bereichs durch Anzeige auf einer Anzeige (62A) ausgibt, und **dadurch gekennzeichnet, dass**
die Benachrichtigungssteuereinheit (58) so konfiguriert ist, dass sie die Anzeigebenachrichtigungseinheit (62) veranlasst, das endoskopische Bild zu überlagern und eine geometrische Form (108) anzuzeigen, die eine Fläche des interessierenden Bereichs an einer Position des interessierenden Bereichs nur zum Zeitpunkt der Erfassung des interessierenden Bereichs im Einführungsschritt anzeigt, und die Anzeigebenachrichtigungseinheit (62) zu veranlassen, das endoskopische Bild zu überlagern und die geometrische Form (108) anzuzeigen, die die Fläche des interessierenden Bereichs an der Position des interessierenden Bereichs zum Zeitpunkt der Erfassung des interessierenden Bereichs anzeigt, und die geometrische Form für eine bestimmte Zeitspanne nach der Erfassung im Entnahmeschritt anzuzeigen.

2. Das Endoskopsystem nach Anspruch 1, wobei
die Benachrichtigungssteuereinheit (58) so konfiguriert ist, dass sie die Anzeigebenachrichtigungseinheit (62) veranlasst, Text (100A) mit einer ersten Größe im Einfügeschritt anzuzeigen, und die Anzeigebenachrichtigungseinheit veranlasst, Text (100B) mit einer zweiten Größe, die größer als die erste Größe ist, im Entnahmeschritt anzuzeigen.

3. Das Endoskopsystem nach Anspruch 1 oder 2, wobei
die Benachrichtigungssteuereinheit (58) so konfiguriert ist, dass sie die Anzeigebenachrichtigungseinheit (60) veranlasst, ein Symbol mit einer ersten Größe in dem Einfügeschritt anzuzeigen, und die Anzeigebenachrichtigungseinheit veranlasst, ein Symbol mit einer zweiten Größe, die größer als die erste Größe ist, in dem Entnahmeschritt anzuzeigen.

4. Das Endoskopsystem nach einem der Ansprüche 1 bis 3, wobei
die Benachrichtigungssteuereinheit (58) so konfiguriert ist, dass sie die Anzeigebenachrichtigungseinheit (60) veranlasst, im Einfügeschritt einen Hintergrund in einer ersten Hintergrundfarbe anzuzeigen, und dass sie die Anzeigebenachrichtigungseinheit veranlasst, im Entnahmeschritt einen Hintergrund in einer zweiten Hintergrundfarbe anzuzeigen, die eine höhere Helligkeit als die erste Hintergrundfarbe aufweist.

5. Das Endoskopsystem nach einem der Ansprüche 1 bis 4, wobei
die Benachrichtigungssteuereinheit (58) so konfiguriert ist, dass sie die Anzeige-Benachrichtigungseinheit (60) veranlasst, einen Rahmen (106A) anzuzeigen, der eine erste Größe hat und den interessierenden Bereich zusammen mit dem endoskopischen Bild im Einführungsschritt enthält, und die Anzeige-Benachrichtigungseinheit veranlasst, einen Rahmen (106B) anzuzeigen, der eine zweite Größe hat, die größer als die erste Größe ist und den interessierenden Bereich zusammen mit dem endoskopischen Bild im Entnahmeschritt enthält.

6. Das Endoskopsystem nach einem der Ansprüche 1 bis 5, wobei
die Detektionsergebnis-Benachrichtigungseinheit (60) eine Ton-Benachrichtigungseinheit (64) umfasst, die so konfiguriert ist, dass sie eine Benachrichtigung über die Detektion des Bereichs von Interesse durch Ausgabe eines Tons gibt, und
die Benachrichtigungssteuereinheit (58) so konfiguriert ist, dass sie die Tonbenachrichtigungseinheit (64) veranlasst, den Ton mit einer ersten Tonlautstärke im Einfügungsschritt auszugeben, und die Tonbenachrichtigungseinheit veranlasst, den Ton mit einer zweiten Tonlautstärke auszugeben, die höher ist als die erste Tonlautstärke im Entnahmeschritt.

7. Das Endoskopsystem nach einem der Ansprüche 1 bis 6, wobei
die Detektionsergebnis-Benachrichtigungseinheit (60) eine Beleuchtungs-Benachrichtigungseinheit (66) umfasst, die so konfiguriert ist, dass sie eine Benachrichtigung über die Detektion des interessierenden Bereichs durch Aufleuchten einer Lampe gibt, und
die Benachrichtigungs-Steuereinheit (58) so konfiguriert ist, dass sie die Beleuchtungs-Benachrichtigungseinheit (66) veranlasst, die Lampe mit einer ersten Lichtmenge im Einführungsschritt zu beleuchten, und die Beleuchtungs-Benachrichtigungseinheit veranlasst, die Lampe mit einer zweiten Lichtmenge, die größer als die erste Lichtmenge ist, im Entnahmeschritt zu beleuchten.

8. Das Endoskopsystem nach Anspruch 1, wobei
Die Detektionsergebnis-Benachrichtigungseinheit (60) umfasst
eine Tonbenachrichtigungseinheit, die so konfiguriert ist, dass sie eine Benachrichtigung durch Ausgabe eines Tons gibt, und
die Benachrichtigungssteuereinheit (58) so konfiguriert ist, dass sie die Tonbenachrichtigungseinheit veranlasst, den Ton im Entnahmeschritt auszugeben.

9. Das Endoskopsystem nach Anspruch 1, wobei
die Detektionsergebnis-Benachrichtigungseinheit (60) umfasst
eine Tonbenachrichtigungseinheit (64) , die so konfiguriert ist, dass sie eine Benachrichtigung durch Ausgabe eines Tons gibt, und
die Benachrichtigungssteuereinheit (58) so konfiguriert ist, dass sie die Tonbenachrichtigungseinheit (64) veranlasst, den Ton im Einfügungsschritt auszugeben, und die Tonbenachrichtigungseinheit veranlasst, den Ton auszugeben, und die Anzeigebenachrichtigungseinheit veranlasst, ein Symbol im Entnahmeschritt anzuzeigen.

10. Das Endoskopsystem nach Anspruch 1, wobei
die Detektionsergebnis-Benachrichtigungseinheit (60) umfasst
eine Tonbenachrichtigungseinheit (64) , die so konfiguriert ist, dass sie eine Benachrichtigung durch Ausgabe eines Tons gibt, und
eine Beleuchtungsmeldeeinheit (66), die so konfiguriert ist, dass sie eine Meldung durch Aufleuchten einer Lampe (66A) gibt, und
die Benachrichtigungs-Steuereinheit (58) so konfiguriert ist, dass sie die Beleuchtungs-Benachrichtigungseinheit (66) veranlasst, die Lampe (66A) in dem Einführungsschritt zu beleuchten, und die Beleuchtungs-Benachrichtigungseinheit (66) veranlasst, die Lampe (66A) zu beleuchten, und die Ton-Benachrichtigungseinheit (64) veranlasst, den Ton in dem Entnahmeschritt auszugeben.

11. Das Endoskopsystem nach Anspruch 1, wobei
die Detektionsergebnis-Benachrichtigungseinheit (60) umfasst
eine Tonbenachrichtigungseinheit (64) , die so konfiguriert ist, dass sie eine Benachrichtigung durch Ausgabe eines Tons gibt, und
eine Beleuchtungsmeldeeinheit (66), die so konfiguriert ist, dass sie eine Meldung durch Aufleuchten einer Lampe (66A) gibt, und
wenn N eine ganze Zahl von 0 bis 2 ist, die Benachrichtigungs-Steuereinheit so konfiguriert ist, dass sie N Einheiten der Anzeige-Benachrichtigungseinheit (62), der Ton-Benachrichtigungseinheit (64) und der Beleuchtungs-Benachrichtigungseinheit (66) veranlasst, in dem Einfügeschritt eine Benachrichtigung zu geben, und veranlasst, dass mindestens (N+1) Einheiten der Anzeige-Benachrichtigungseinheit, der Ton-Benachrichtigungseinheit und der Beleuchtungs-Benachrichtigungseinheit in dem Entnahmeschritt eine Benachrichtigung geben.

## Revendications

1. Système d'endoscope (10) pour effectuer un examen de la lumière d'un patient, le système d'endoscope comprenant :
une partie d'insertion (12A) configurée pour être insérée dans la lumière ;
une caméra configurée pour effectuer une capture d'image de la lumière afin d'obtenir une image endoscopique ;
une unité de détection de région d'intérêt (50) configurée pour détecter une région d'intérêt de l'image endoscopique ;
une unité de notification du résultat de la détection (60) configurée pour donner à une notification du résultat de la détection de la région d'intérêt ;
une unité de détermination de l'insertion/retrait (68) configurée pour déterminer si une étape de l'examen est une étape d'insertion au cours de laquelle la pièce d'insertion est insérée jusqu'à un point de retour dans la lumière ou une étape de retrait au cours de laquelle la pièce d'insertion est retirée du point de retour ; et
une unité de commande de notification (58) configurée pour faire en sorte que l'unité de notification du résultat de la détection émette la notification conformément à l'étape déterminée par l'unité de détermination de l'insertion-retrait ; dans laquelle
l'unité de notification du résultat de la détection (60) comprend une unité de notification d'affichage (62) configurée pour donner une notification de détection de la région d'intérêt par affichage sur un écran (62A), et **caractérisée en ce que**
l'unité de commande de notification (58) est configurée pour amener l'unité de notification d'affichage (62) à superposer sur l'image endoscopique et à afficher une forme géométrique (108) qui indique une zone de la région d'intérêt à une position de la région d'intérêt uniquement au moment de la détection de la région d'intérêt au cours de l'étape d'insertion, et de faire en sorte que l'unité de notification d'affichage (62) superpose à l'image endoscopique et affiche la forme géométrique (108) qui indique la zone de la région d'intérêt à la position de la région d'intérêt au moment de la détection de la région d'intérêt et continue d'afficher la forme géométrique pendant une certaine période après la détection lors de l'étape de retrait.

2. Système d'endoscope selon la revendication 1, dans lequel
l'unité de commande de notification (58) est configurée pour faire en sorte que l'unité de notification d'affichage (62) affiche un texte (100A) d'une première taille lors de l'étape d'insertion, et pour faire en sorte que l'unité de notification d'affichage affiche un texte (100B) d'une seconde taille supérieure à la première lors de l'étape de retrait.

3. Système d'endoscope selon la revendication 1 ou 2, dans lequel
l'unité de commande de notification (58) est configurée pour faire en sorte que l'unité de notification d'affichage (60) affiche une icône ayant une première taille lors de l'étape d'insertion, et pour faire en sorte que l'unité de notification d'affichage affiche une icône ayant une seconde taille plus grande que la première lors de l'étape de retrait.

4. Système d'endoscope selon l'une des revendications 1 à 3, dans lequel
l'unité de commande de notification (58) est configurée pour faire en sorte que l'unité de notification d'affichage (60) affiche un arrière-plan dans une première couleur d'arrière-plan lors de l'étape d'insertion, et pour faire en sorte que l'unité de notification d'affichage affiche un arrière-plan dans une deuxième couleur d'arrière-plan plus lumineuse que la première couleur d'arrière-plan lors de l'étape de retrait.

5. Système d'endoscope selon l'une des revendications 1 à 4, dans lequel
l'unité de commande de notification (58) est configurée pour amener l'unité de notification d'affichage (60) à afficher un cadre (106A) ayant une première taille et comprenant la région d'intérêt avec l'image endoscopique dans l'étape d'insertion, et pour amener l'unité de notification d'affichage à afficher un cadre (106B) ayant une seconde taille plus grande que la première taille et comprenant la région d'intérêt avec l'image endoscopique dans l'étape de retrait.

6. Système d'endoscope selon l'une des revendications 1 à 5, dans lequel
l'unité de notification du résultat de la détection (60) comprend une unité de notification sonore (64) configurée pour donner à une notification de détection de la région d'intérêt en émettant un son, et
l'unité de commande de notification (58) est configurée pour que l'unité de notification sonore (64) émette le son à un premier volume sonore lors de l'étape d'insertion, et pour que l'unité de notification sonore émette le son à un second volume sonore supérieur au premier volume sonore lors de l'étape de retrait.

7. Système d'endoscope selon l'une des revendications 1 à 6, dans lequel
l'unité de notification du résultat de la détection (60) comprend une unité de notification de l'éclairage (66) configurée pour donner à une notification de la détection de la région d'intérêt par l'allumage d'une lampe, et
l'unité de commande de notification (58) est configurée pour faire en sorte que l'unité de notification d'éclairage (66) allume la lampe avec une première quantité de lumière lors de l'étape d'insertion, et pour faire en sorte que l'unité de notification d'éclairage allume la lampe avec une deuxième quantité de lumière supérieure à la première quantité de lumière lors de l'étape de retrait.

8. Système d'endoscope selon la revendication 1, dans lequel
l'unité de notification du résultat de la détection (60) comprend
une unité de notification sonore configurée pour donner à une notification en émettant un son, et
l'unité de commande de notification (58) est configurée pour faire en sorte que l'unité de notification sonore émette le son lors de l'étape de retrait.

9. Système d'endoscope selon la revendication 1, dans lequel
l'unité de notification du résultat de la détection (60) comprend
une unité de notification sonore (64) configurée pour donner à une notification en émettant un son, et
l'unité de commande de notification (58) est configurée pour faire en sorte que l'unité de notification sonore (64) émette le son lors de l'étape d'insertion, et que l'unité de notification sonore émette le son et que l'unité de notification d'affichage affiche une icône lors de l'étape de retrait.

10. Système d'endoscope selon la revendication 1, dans lequel
l'unité de notification du résultat de la détection (60) comprend
une unité de notification sonore (64) configurée pour donner à une notification en émettant un son, et
une unité de notification d'éclairage (66) configurée pour donner à une notification en allumant une lampe (66A), et
l'unité de commande de notification (58) est configurée pour faire en sorte que l'unité de notification d'éclairage (66) allume la lampe (66A) lors de l'étape d'insertion, et fait en sorte que l'unité de notification d'éclairage (66) allume la lampe (66A), et pour faire en sorte que l'unité de notification de son (64) émette le son lors de l'étape de retrait.

11. Système d'endoscope selon la revendication 1, dans lequel
l'unité de notification du résultat de la détection (60) comprend
une unité de notification sonore (64) configurée pour donner à une notification en émettant un son, et
une unité de notification d'éclairage (66) configurée pour donner à une notification en allumant une lampe (66A), et
lorsque N est un nombre entier compris entre 0 et 2, l'unité de commande de notification est configurée pour que N unités parmi l'unité de notification de l'affichage (62), l'unité de notification du son (64) et l'unité de notification de l'éclairage (66) émettent une notification lors de l'étape d'insertion, et pour qu'au moins (N+1) unités parmi l'unité de notification de l'affichage, l'unité de notification du son et l'unité de notification de l'éclairage émettent une notification lors de l'étape de retrait.
